# EUROPEAN PATENT APPLICATION

(11) **EP 2 417 934 A1**
(43) Date of publication of application: **15.02.2012**
(21) Application number: 10425271.3
(22) Date of filing: 10.08.2010
(51) Int. Cl.: A61F 2/24

(54) **Stent-based extra-venous support for venous valve repair**

(71) Applicant: SANGOMED S.R.L., 00139 Rome (IT)
(72) Inventor: Camilli, Sante, 00187 Roma (RM) (IT); Camilli, Daniele, 00139 Roma (RM) (IT)

(57) **Abstract**

An oval-shaped extra-venous support for venous valve repair, including a cylindrical channel mesh shell, the cross-section whereof is oval, which is a one-piece one (1), laterally open (g) and includes anchor means (101', 101", 101"'; 103'; 103", 103"') in correspondence with at least a cross-section thereof at both the opposite extremities of the major axis thereof. Such major axis is made longer than the intercommissural diameter of an incompetent native valve to be cured, so that it is capable of operating as a traction-acting device in cooperation with fastening means to stretch the intercommissural walls of the valve so as to extend the free edges (C1, C2) of the cusps thereof up to recover the capability thereof of coapting with each other.

## Description

### TECHNICAL FIELD

This invention relates to the field of Vascular Surgery.

More particularly, this invention relates to a stent-based extra-venous support for treating the incompetence of venous valves according to the preamble of Claim 1.

By 'stent' it is generally meant herein a device having a mesh structure composed of mesh branches made of an elastically flexible elongate material, suitable for implantation in or about an anatomical vessel, as is known in the Surgery art.

By 'elongate material' a material is meant herein in a shape having its thickness and its width small as compared to its length, such as e.g. a wire or a strip material.

The elastically flexible material may be e.g. a metal, a plastic material or an alloy.

### BACKGROUND OF THE INVENTION

Venous valves are to allow centripetal (i.e. towards the heart) unidirectional blood flow. They are formed by a reduplication of the inner venous layer, which forms two semilunar valve cusps, attached by an edge thereof to the venous wall, while the respective other edges are free edges, opposite to each other, which are freely moving in the vein lumen. The valve cusps lie in close apposition with the venous wall as long as the blood flow takes its natural course. If any regurgitation takes place, the cusps become distended, their opposite free edges are brought to coapt into reciprocal contact and the reversal blood flow is interrupted.

A characteristic of a venous valve is its intercommissural diameter, along which the opposite free edges of the cusps go to coapt. The intercommissural diameter has intercommissural extremities.

The valvular incompetence of a vein consists in a slackening of the free edge of the valve cusps. Such a slackening involves an incapability of the free edges of the cusps to coapt, so that the unidirectional function of the valves is compromised, unduly allowing the reversal of the centripetal flow of venous blood.

The Publication of European Patent Application No. 1,561,437 (Inventor SANTE CAMILLI) of 10^{th} August, 2005, having the title *"External Support for Restoring Competence to Venous Valves by Traction of Their Intercommissural Walls",* referred to as Document D1 hereinafter, is the closest prior art document, the description whereof is fully incorporated herein as a matter of reference. D1 discloses an external or extra-venous stent-based support for treating the incompetence of venous valves. By 'external' or 'extra-venous' it is meant that the support is to be implanted externally to a vein.

The external support of D1 has a stent mesh structure. It is composed of two separate mesh shell valves, to be closed the one onto the other by hinging them in correspondence with two opposite longitudinal sides thereof, forming a tubular 'shell' construction. Each shell valve is endowed with two eyelets on a longitudinal side thereof and with two free-end branches on the opposite longitudinal side thereof. The two shell valves are hinged to each other by inserting the free-end branches of a shell valve into the eyelets of the other shell valve.

The external support of D1 is a flattened generally cylindrical support featuring an oval-shaped support (by 'oval-shaped' it is meant herein a general channel shape the cross-section whereof is an oval, i.e. a closed plane curve that delimits a convex region of the plane, having a major symmetry axis; or an arc of an oval. Such an oval may be an ellipse or a circle as particular cases).

The support of D1 is to be implanted by closing its two shell valves about a vein, in correspondence with an incompetent valve thereof. Once implanted, it is suitable for curing the incompetence of the venous valve by cooperating with means for fastening it to the venous wall. Particularly, it is envisaged to use surgical suture stitches. The latter become effective by respectively engaging the venous wall - in correspondence with the intercommissural walls, in the proximity of the opposite extremities of the intercommissural diameter of the venous valve - and the mesh branches of the support in correspondence with the opposite longitudinal sides thereof. A traction force arises of the suture stitches on the venous wall by virtue of the major axis of the oval-shaped support being greater than the intercommissural diameter of the native venous valve. This traction force stretches the intercommissural walls apart dilating the intercommissural diameter of the incompetent valve, so extending the slackened free edges of the valve cusps recovering the capability thereof of coapting with each other.

So the external support of D1 is suitable for recovering the function of the edges of the cusps of coapting with one another by virtue of an intercommissural stretching which can be realized by means of it.

Though being effective, the support of D1 has disadvantages, consequent on its constitution in two pieces to be assembled in *situ.* The disadvantages concern the manufacture and the utilization thereof. From the standpoint of its manufacturing, the two-piece constitution thereof does not allow the possibilities to be fully exploited of both ablative top-down technologies, including laser-cutting starting from a tube shell or a sheet, and bottom-up technologies of continuous-wire modelling and plastic casting.

From the standpoint of its utilization, the two-piece constitution thereof involves the complication that it requires its two shell valves to be assembled *in situ.* Moreover, the free-end branches thereof constitute tip-ended members which can injure the venous wall or other surrounding body structures.

### DISCLOSURE OF THE INVENTION

Therefore, it is the object of this invention to improve an oval-shaped external or extra-venous stent-based support for venous valve repair for implantation about an incompetent venous valve, the support including an oval-shaped channel shell composed of mesh branches made of an elastically flexible elongate material, and being capable of operating as a traction-acting device in cooperation with fastening means engaging it to the wall of the vein in correspondence with the opposite intercommissural walls of the incompetent valve thereof to stretch them apart in order to dilate the intercommissural diameter of the incompetent valve so as to extend the slackened free edges of the cusps thereof up to recover the capability thereof of coapting with each other.

The improvement taught by this invention consists in that the shell of the support is a one-piece one. The channel shape of the latter is laterally open, i.e. it has a continuous opening running all along its lateral surface. This clearly allows the support to be arranged externally about a vein.

The inventive support is endowed with preceptive anchor means in correspondence of at least a cross-section thereof at both said opposite extremities of the major axis of the oval shape of the latter. It is envisaged that the anchor means are eyelets capable of being engaged to the wall of a vein by fastening means, such as surgical suture stitches.

There are substantial differences between the support of this invention and the support of D1.

The support of this invention is a one-piece one as opposed to the support of D1, which is a two-piece one.

The eyelets as anchor means in the support of this invention are for engaging the support to a venous wall, whilst the eyelets in the support of D1 are for assembling the two pieces thereof together, and are not suitable for the intercommissural stretching, because the eyelets are provided on a side only of each shell valve of the support, not by both the opposite sides of it as in the support of this invention, which is necessary to make engaging means available in correspondence with both the opposite intercommissural walls of a venous valve to dilate its intercommissural diameter.

It is envisaged in D1 to provide the external support taught therein with hooks, but these ones are merely additional means not able to perform the main function per se of engaging the support to a vein wall in a precise and surely stable way.

Therefore it is the subject-matter of this invention a traction-acting oval-shaped external or extra-venous support for venous valve repair according to Claim 1, for implantation about a vein having venous valves thereinside having cusps with respective opposite free edges that are to coapt along a venous valve intercommissural diameter having intercommissural extremities attached to respective opposite intercommissural walls of the venous valve, in correspondence with a venous valve the cusp free edges whereof have become slackened whereby they have lost their ability to coapt with each other, so that the venous valve has become incompetent; the extra-venous support including a mesh shell composed of mesh branches made of an elastically flexible elongate material and having the general shape of a cylindrical channel shell having generatrices, and the cross-section whereof is oval with a major axis having opposite extremities, so the mesh shell being an oval-shaped cylindrical channel mesh shell; the external or extra-venous support being capable of operating as a traction-acting device in cooperation with fastening means engaging it to the wall of the vein in correspondence with the opposite intercommissural walls of the incompetent valve thereof to stretch them apart in order to dilate the intercommissural diameter of the incompetent valve so as to extend the slackened free edges of the cusps thereof up to recover the capability thereof of coapting with each other; the oval-shaped cylindrical channel mesh shell being a one-piece one, laterally open and including anchor means in correspondence of at least a cross-section thereof at both said opposite extremities of the major axis of the cross-section itself.

Preferred embodiments are set forth in dependant Claims 2 to 10.

### BRIEF DESCRIPTION OF THE DRAWINGS

This invention will be understood based on the following detailed description of preferred embodiments thereof, only given as a matter of example, absolutely not of restriction, of the teaching thereof, referring to the enclosed drawings, wherein:
- FIGURE 1 is a spatial perspective depictive view (without a rigorous respect of the geometrical ratios between the portions) of the inventive extra-venous support according to a first embodiment;
- FIGURE 1A shows the inventive extra-venous support of FIGURE 1 developed on the plane;
- FIGURES 2 and 3 show the inventive extra-venous support respectively according to a second and a third embodiment, developed on the plane;
- FIGURE 3A is a schematic functional depiction of a cross-section view of a venous valve whereabout an extra-venous support of FIGURES 1 and 1A or of FIGURE 2 or 3 has been implanted;
- FIGURE 4 shows the inventive extra-venous support according to a fourth embodiment, developed on the plane;
- FIGURE 4A is a schematic functional depiction of a cross-section view of a venous valve whereabout the extra-venous support of FIGURE 4 has been implanted;
- FIGURE 5 shows the inventive extra-venous support according to a fifth embodiment, developed on the plane;
- FIGURE 5A is a schematic functional depiction of a cross-section view of a venous valve whereabout the extra-venous support of FIGURE 5 has been implanted;
- FIGURE 6 shows the inventive extra-venous support according to a sixth embodiment, developed on the plane;
- FIGURE 7 shows the inventive extra-venous support according to a seventh embodiment implanted about a vein, in a front view;
- FIGURE 7A shows the extra-venous support of FIGURE 8 implanted about a vein in a side view, and
- FIGURE 7B shows a schematic cross-section view of a venous valve whereabout an extra-venous support of FIGURE 6 or of FIGURES 7 and 7A has been implanted.

### BEST WAYS FOR CARRYING OUT THE INVENTION

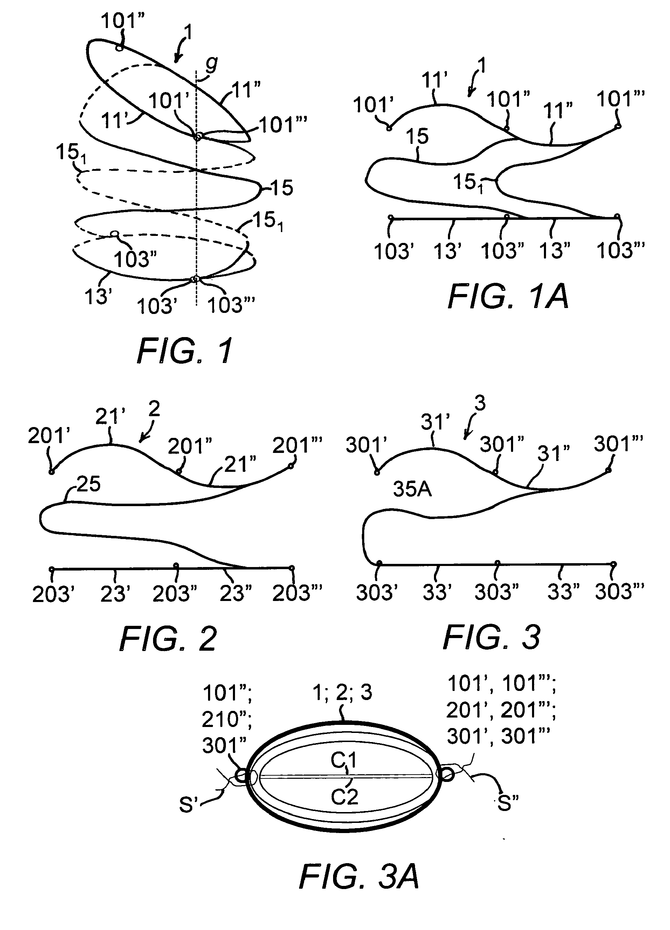

Referring to FIGURES 1 and 1A, according to a first preferred embodiment of the inventive extra-venous support, oval-shaped cylindrical mesh shell 1 includes an upper 11'-11" and a lower 13'-13" generally oval ring, which have respective major axes having extremities. The rings are each one open with two free ends in correspondence of an extremity of the respective major axis thereof along the same generatrix g of cylindrical channel mesh shell 1. The free ends are endowed with eyelets as anchor means in correspondence with two free ends 101', 101"'; 103'; 103"' and with the opposite major axis extremity 101"; 103" of each one of rings 11'-11"; 13'-13". The support includes two generally bell-shaped connecting bends 15, 15₁ which constitute means for mechanically connecting upper ring 11'-11" and lower ring 13'-13" together. Connecting bends 15, 15₁ are equally oriented and connect the rings at intermediate points thereof.

Referring to FIGURE 2, according to a second embodiment the inventive support includes one generally bell-shaped connecting bend 25 as means for mechanically connecting upper ring 21'-21" and lower ring 23'-23". Connecting bends 25 connects the rings at intermediate points thereof.

Referring to FIGURE 3, according to a third embodiment the inventive support includes one connecting bend in the general shape of an incomplete bell 35A as means for mechanically connecting upper ring 31'-31" and lower ring 33'-33". Connecting bend 35A connects the rings at an intermediate point of upper ring 31'-31" and at a free end of lower ring 33'-33".

FIGURE 3A equally depicts the implantation about a vein V of inventive support 1, 2 or 3 respectively according to the first, the second and the third embodiment thereof, fastened to vein wall V by eyelets 101', 101"' and 101"; 201', 201"' and 201" or 301', 301"' and 301" respectively.

Referring to FIGURE 3A, the inventive support according to the first to the third embodiment is implanted about a vein V with intermediate eyelets 101", 103" in correspondence with an intercommissural wall in proximity with an intercommissural extremity of an incompetent valve and with diametrically opposite extreme eyelets 101', 101"'; 103', 103"' in correspondence with the opposite intercommissural wall in the proximity of the opposite intercommissural extremity of the incompetent native valve. Support 1; 2; 3 envelops vein V fully.

Surgical suture stitches S', S" are made to fasten support 1; 2; 3 to the wall of vein V, solidarizing it thereto, respectively engaging an intercommissural wall and intermediate eyelet 101"; 201"; 301" and the opposite intercommissural wall and extreme eyelets 101', 101"'; 201', 201"'; 301', 301"'.

The major axis of the generally oval rings is made greater than the intercommissural diameter of the incompetent native valve. So, in cooperation with surgical suture stitches S', S" support 1; 2; 3 is capable of operating as a traction-acting device stretching the intercommissural walls of an incompetent native valve apart in order to dilate the intercommissural diameter of the incompetent valve so as to extend free edges C1, C2 of the cusps thereof up to recover the capability thereof of coapting with each other.

It is envisaged that a ring, for instance upper ring 11'-11", as can be seen in FIGURE 1, is slanted with respect to the longitudinal direction of the support. This slanting can be seen in FIGURES 1A, 2 and 3, where upper ring 11'-11"; 21'-21" or 31'-31" has a curve planar development having a flex featuring an upper arc 11', 21', 31' turning a convexity and respectively an upper arc 11", 21", 31" turning a concavity towards outside the support. This embodiment facilitates the implantation of the support, particularly in correspondence with venous confluences.

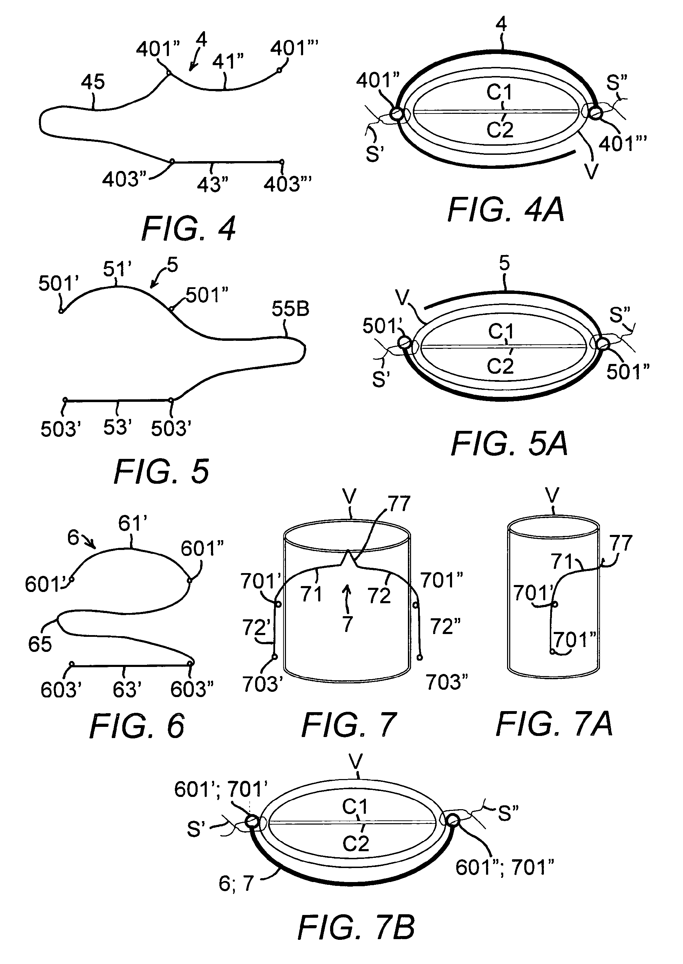

Referring to FIGURES 4, 5 and 6, according to a fourth to a sixth embodiment of the inventive extra-venous support, oval-shaped cylindrical mesh shell 4; 5; 6 includes an upper 41"; 51'; 61' and a lower 43"; 53'; 63' half-ring having the general shape of a half-oval insisting on the major axis thereof respectively having two free ends endowed with eyelets 401", 401"', 403", 403"'; 501', 501"; 503', 503"; 601', 601"; 603', 603" as anchor means in correspondence with the extremities of the major axes thereof.

Referring to FIGURE 4, according to a fourth embodiment upper half-ring 41" and lower half-ring 43" are connected by extremities 401", 403" thereof by generally bell-shaped bend 45, which develops to partially envelop a tubular volume along with the upper 41" and lower 43" half-ring. So, as depicted in FIGURE 4A, the support in this fourth embodiment once implanted about vein V incompletely envelops the latter.

Referring to FIGURE 5, according to a fifth embodiment upper half-ring 51' and lower half-ring 53' are connected by extremities 501", 503" thereof by generally bell-shaped bend 55B, which develops to partially envelop a tubular volume along with upper 51' and lower 53' half-ring. So, as depicted in FIGURE 5A, the support in this fifth embodiment once implanted about vein V incompletely envelops the latter.

Referring to FIGURE 6, according to a sixth embodiment upper half-ring 61' and lower half-ring 63' are mechanically connected by extremities 601", 603" thereof by generally bell-shaped bend 65, which develops to envelop a half-tubular volume together with upper 61' and lower 63' half-ring. So, as depicted in FIGURE 7B, the support in this sixth embodiment once implanted about vein V envelops the latter by half.

It is envisaged that a half-ring, for instance the upper half-ring, is slanted with respect to the longitudinal direction of the support. This can be seen in FIGURE 4, where upper half-ring 41" developed on the plane turns a concavity towards outside the support, and in FIGURES 5 and 6, where upper half-rings 51'; 61' developed on the plane turn a convexity towards outside the support. This is suitable to facilitate the implantation of the support, particularly in correspondence with venous confluences.

Referring to FIGURES 7 and 7A, according to a seventh embodiment, the one-piece laterally open oval-shaped channel mesh shell includes a splay central arc 71 spatially bent conformingly to the profile of a cylindrical surface the cross-section whereof is a half-oval insisting on the major axis thereof and extended beyond both the extremities 701', 701" thereof by two side extension arcs 72', 72", respectively having a free extremity and an internal extremity in common with splay central arc 71. Support 7 is endowed with eyelets 701', 703'; 701", 703" as anchor means in correspondence with the extremities of the central arc and of the extension arcs. As depicted in FIGURE 7B, the support in this seventh embodiment once implanted about vein V partially envelops the latter by half.

Splay central arc 71 may include a bulging portion suitable for facilitating the introduction of support 7 into a catheter-based delivery system. As depicted in FIGURE 7A the bulging portion may be a tip portion 77. It is also envisaged, however, that it may have other shapes, e.g. a horseshoe shape.

The seventh embodiment is advantageous in that it is capable of being folded into a small tubular space of a catheter-based delivery system which is possible to use with minimally invasive video-assisted procedures.

The implantation of the inventive support according to the fourth to the seventh embodiment is performed by surgical suture stitches S', S" which are made to fasten support 4; 5; 6; 7 to the wall of vein V, solidarizing it thereto, respectively engaging an intercommissural wall and an eyelet 401"; 501'; 601'; 701' and the opposite intercommissural wall and opposite eyelet 401"'; 501"; 601"; 701".

The major axis of the support is made greater than the intercommissural diameter of the incompetent native valve. So, in cooperation with surgical suture stitches S', S" support 4; 5; 6; 7 is capable of operating as a traction-acting device for recovering the capability of free edges C1, C2 of the valve cusps of coapting with each other as expounded above.

The extra-venous support of this invention may be manufactured in any material which exhibits elastic flexibility, such as e.g. an elastic metal alloy, including medical grade steel, or a superelastic metal alloy, including Nitinol®; or in a plastic material, particularly superelastic polymers.

The embodiments disclosed above are more or less suitable depending on the preferred process for manufacturing them and/or on the type of the utilization thereof. They have different geometrical and mechanical characteristics and, consequently, a diversified applicability and handling, with the object of giving the surgeon the possibility of adaptation to different anatomical and clinical situations.

This invention has only been disclosed and depicted referring to preferred embodiments thereof as a matter of example, absolutely not of restriction of the original teaching thereof, but it is to be expressly understood that variations, omissions or additions may be made without departing from the relevant scope of protection, which only remains defined by the appended claims.

For instance, the oval-shaped cylindrical channel mesh shell may reduce to a generally oval open ring or to a half-ring having the general shape of a half-oval insisting on the major axis thereof.

The inventive device as disclosed includes eyelets, but it may include other anchor means, such as e.g. hooks.

## Claims

1. An oval-shaped external or extra-venous support for venous valve repair, for implantation about a vein (V) having venous valves thereinside having cusps with respective opposite free edges (C1, C2) that are to coapt along a venous valve intercommissural diameter having intercommissural extremities attached to respective opposite intercommissural walls of the venous valve, in correspondence with a venous valve the cusp free edges whereof have become slackened whereby they have lost their capability to coapt with each other, so that said venous valve has become incompetent; the extra-venous support including a mesh shell composed of mesh branches made of an elastically flexible elongate material and having the general shape of a cylindrical channel shell having generatrices, and the cross-section whereof is oval with a major axis having opposite extremities, so that the mesh shell is an oval-shaped cylindrical channel mesh shell; the external or extra-venous support being capable of operating as a traction-acting device in cooperation with fastening means engaging it to the wall of the vein (V) in correspondence with the opposite intercommissural walls of the incompetent valve thereof to stretch them apart in order to dilate the intercommissural diameter of the incompetent valve so as to extend the slackened free edges (C1, C2) of the cusps thereof up to recover the capability thereof of coapting with each other;
**characterized in that** said oval-shaped cylindrical channel mesh shell is a one-piece one (1; 2; 3; 4; 5; 6; 7), laterally open (g) and includes anchor means (101', 101", 101"'; 103'; 103", 103"'; 201', 201", 201"'; 203'; 203", 203"'; 301', 301", 301"'; 303'; 303", 303"'; 401", 401"', 403", 403"'; 501', 501"; 503', 503"; 601', 601"; 603', 603" 701', 701"; 703', 703") in correspondence of at least a cross-section thereof at both said opposite extremities of the major axis of the cross-section itself.

2. The oval-shaped external or extra-venous support for venous valve repair according to Claim 1, wherein said anchor means are eyelets capable of being engaged to the wall of a vein (V) by fastening means (S', S").

3. The oval-shaped external or extra-venous support according to Claim 1, wherein said laterally open one-piece oval-shaped cylindrical channel mesh shell includes (1; 2; 3) an upper (11'-11"; 21'-21"; 31'-31") and a lower (13'-13"; 23'-23"; 33'-33") generally oval ring having respective major axes having extremities, each one open with two free ends in correspondence of an extremity of the respective major axis thereof along the same generatrix (g) of said cylindrical channel mesh shell (1); and endowed with anchor means in correspondence with said two free ends (101', 101"'; 103'; 103"') and with the opposite extremity of said major axis (101"; 103") of each one of said rings; and including at least one generally bell-shaped connecting bend (15, 15₁; 25; 35A) mechanically connecting said upper and lower ring together.

4. The oval-shaped external or extra-venous support according to Claim 3, wherein a ring (11'-11"; 21'-21"; 31'-31") is slanted with respect to the longitudinal direction of the support (1; 2; 3).

5. The oval-shaped external or extra-venous support according to Claim 1, wherein said laterally open one-piece oval-shaped cylindrical channel mesh shell includes (4; 5; 6) an upper (41"; 51'; 61') and a lower (43"; 53'; 63') half-ring having the general shape of a half-oval insisting on the major axis thereof respectively having two free ends endowed with anchor means (401", 401"', 403", 403"'; 501', 501"; 503', 503"; 601', 601"; 603', 603"), in correspondence with the extremities of the major axes thereof; and including at least one generally bell-shaped connecting bend (45; 55B; 65) mechanically connecting said upper (41"; 51'; 61') and lower (43"; 53'; 63') half-rings together.

6. The oval-shaped external or extra-venous support according to Claim 5, wherein a half-ring (41"; 51'; 61') is slanted with respect to the longitudinal direction of the support.

7. The oval-shaped external or extra-venous support according to Claim 1, wherein said laterally open one-piece oval-shaped channel mesh shell includes (7) a splay central arc (71) having extremities (701', 701") spatially bent conformingly to the profile of a cylindrical surface the cross-section whereof is a half-oval insisting on the major axis thereof, and extended beyond both said extremities (701', 701") thereof by two side extension arcs (72', 72"), respectively having a free extremity and an extremity in common with said splay central arc (71); the support (7) including anchor means (701', 701"; 703', 703") in correspondence with said extremities of said extension arcs (72', 72").

8. The oval-shaped external or extra-venous support according to Claim 7, wherein said splay central arc (71) includes a bulging portion (77), suitable for facilitating the introduction of the support (7) into a catheter-based delivery system.

9. The oval-shaped external or extra-venous support according to Claim 1, wherein said oval-shaped cylindrical channel mesh shell reduces to a generally oval ring having a major axis having extremities, open with two free ends in correspondence of an extremity of said major axis; and endowed with anchor means in correspondence with said two free ends and with the opposite extremity of said major axis.

10. The oval-shaped external or extra-venous support according to Claim 1, wherein said oval-shaped cylindrical channel mesh shell reduces to a half-ring having the general shape of a half-oval insisting on the major axis thereof having two free ends, endowed with anchor means.
